# EUROPÄISCHE PATENTSCHRIFT

(11) **EP 1 696 832 B1**
(45) Veröffentlichungstag und Bekanntmachung des Hinweises auf die Patenterteilung: **01.08.2007**
(21) Anmeldenummer: 05766824.6
(22) Anmeldetag: 11.05.2005
(51) Int. Cl.: A61F 2/16

(54) **KAPSELÄQUATORRING**
CAPSULAR EQUATORIAL RING
ANNEAU EQUATORIAL CAPSULAIRE

(30) Priorität: 03.06.2004 DE 102004027236
(43) Veröffentlichungstag der Anmeldung: 06.09.2006
(73) Patentinhaber: Morcher GmbH, 70374 Stuttgart (DE)
(72) Erfinder: DICK, Burkhard, 55130 Mainz (DE); MORCHER, Olaf, 70374 Stuttgart (DE)
(74) Vertreter: Patentanwalts-Partnerschaft Rotermund + Pfusch + Bernhard
(86) Internationale Anmeldenummer: PCT/EP2005/052131
(87) Internationale Veröffentlichungsnummer: WO 2005/117748

(56) Entgegenhaltungen:
- EP-A- 0 496 022
- EP-A- 0 507 292
- WO-A-20/04069101
- FR-A- 2 754 173
- US-B1- 6 319 282

## Beschreibung

Die Erfindung betrifft einen Kapseläquatorring, welcher nach dem Entfernen einer natürlichen Linse in den eröffneten Kapselsack eines Auges implantierbar ist und im implantierten Zustand mit seinem Außenumfang an der Innenseite des Kapselsacks im wesentlichen an dessen Äquator anliegt und den Kapselsack radial stabilisiert.

Kapselsackäquatorringe dienen zur Stabilisierung des Kapselsacks im Auge. Sie werden als Implantat in den intakten Kapselsack eingesetzt und dienen, beispielsweise nach Entfernen der natürlichen Linse eines Auges der Stützung des Kapselgewebes. Nach Entfernen der natürlichen Linse, beispielsweise aufgrund einer starken Trübung, ist es erforderlich, dass der eröffnete Kapselsack im wesentlichen in seiner ursprünglichen Form verbleibt und dadurch die Implantation einer künstlichen intraokularen Linse erleichtert werden kann. Bei der Kataraktoperation kann jedoch beim Entfernen der natürlichen Linse das Zonulafasergewebe verletzt werden, welches den Kapselsack im Bereich seines Äquators außenseitig innerhalb des Auges haltert. Um die damit verbundenen Verformungen des Kapselsacks bzw. übermäßige Beanspruchungen der unbeschädigt gebliebenen Zonulafasern zu vermeiden, ist es aus der FR-A 2754173 bekannt, im eröffneten Kapselsack einen Kapseläquatorring der eingangs angegebenen Art zu implantieren. Der Kapseläquatorring verbleibt bei der Operation und in der Regel auch nach dem Einsatz einer Intraokularlinse im Kapselsack und drückt gegen das ihn ringförmig umgebende Gewebe. Aus der FR-A 2754173 ist desweiteren bekannt, dass ein Kapseläquatorring zumindest teilweise aus wasseraufnahmefähigem Material, insbesondere JJEMA/MJ JMA Copolymer bestehen und mit einem wässrigen bzw. wasserlöslichen Medikament getränkt sein kann.

Allgemein können folgende Indikationen für die Implantation eines Kapseläquatorringes im Kapselsack angegeben werden:
- Lokal fehlende oder beschädigte Zonulafasern,
- Gewährleistung beständiger Operationsbedingungen,
- Luxation einer intraokularen Linse (IOL),
- wünschenswerte Ausdehnung bzw. Ausspannung des Kapselsackes,
- Stabilisierung des Kapselsackes nach Entfernung der Linse bei hoher Myopie,
- Zonulolyse,
- Pseudoexfolation,
- Marchesani-Syndrom und
- vereinfachte Implantation faltbarer intraokularer Linsen.

Im übrigen ergeben sich aus der Implantation des Kapseläquatorringes folgende Vorteile:
- Zirkuläre Ausspannung des Kapselsacks,
- beständige Operationsbedingungen,
- Nachstarprophylaxe,
- Hemmung der Kapselsackschrumpfung,
- Minimierung bzw. Vermeidung von Kapselsackfalten,
- Reduzierung der Eintrübung des Vorderkapselrandes und dadurch bessere Fundusvisualisierung, z.B. bei Patienten mit Netzhautproblemen.

Aus der EP 0 507 292 A1 ist ein geschlossener Kapseläquatorring bekannt, welcher nach dem Entfernen einer natürlichen Linse in den eröffneten Kapselsack eines Auges implantierbar ist. Der geschlossen ausgebildete Kapseläquatorring weist dabei vorzugsweise einen rechteckigen oder kreisförmigen Querschnitt auf, wobei bei einer bevorzugten Ausführungsform an einem Innenumfang des Kapseläquatorrings eine Aufnahmenut vorgesehen ist, in welche eine später implantierte Intraokularlinse eingreift und dadurch gehaltert ist. Prinzipiell ist dabei der Kapseläquatorring einstückig ausgebildet.

Aus der DE 197 24 108 C1 ist ein Kapseläquatorring bekannt, welcher als C-förmiger, offener, elastischer Federbügel mit nach einwärts abgebogenen Enden ausgebildet ist, die im implantierten Zustand des Kapseläquatorrings gegen Federwiderstand derart aneinander angenähert sind, dass sich der Kapseläquatorring im implantierten Zustand aufzuweiten sucht. Der Kapseläquatorring kann sich dadurch einerseits aufgrund seiner Federbügelcharakteristik der Größe des jeweiligen Kapselsackes ohne weiteres anpassen und vermag sich andererseits mit merklicher Spannung an den Äquator des Kapselsackes anzulegen.

Aus der DE 199 51 148 A1 ist ein Kapselspannring mit einem bogenförmigen elastischen Element bekannt, wobei die Enden des elastischen Elementes miteinander in Verbindung stehen und vorzugsweise überlappen. Hierdurch wird erreicht, dass der Kapselspannring zumindest im eingesetzten und gespannten Zustand einen Winkel von mehr als 360° umschließt und dadurch auf einer vollständigen Kreislinie von außen einwirkenden Gewebekräften entgegenwirkt.

Aus der DE 202 06 342 U1 ist ebenfalls ein Kapselspannring mit einem elastischen Element bekannt, das im eingesetzten Zustand eine geschlossene Form besitzt. Um die im eingesetzten Zustand geschlossene Form zu erzeugen, weist jedes Ende des elastischen Elementes einen Kopf auf, von denen ein erster Kopf, eine von dem Element fortweisende Aufnahme und der zweite Kopf eine von dem Element fortweisende Zunge besitzt, die derart zueinander angeordnet sind, dass bei einer Kraft in radialer Richtung auf das Element die Zunge in die Aufnahme eintritt und an den Grund der Aufnahme anliegt.

Die vorliegende Erfindung beschäftigt sich mit dem Problem, für einen Kapseläquatorring eingangs erwähnter Art eine verbesserte Ausführungsform anzugeben, bei welcher insbesondere eine zuverlässige Ausspannung des Kapselsackes bei gleichzeitig geringer Belastung des Zonulagewebes während und nach der Implantation gewährleistet ist.

Dieses Problem wird erfindungsgemäß durch den Gegenstand des unabhängigen Anspruchs gelöst. Vorteilhafte Ausführungsformen sind Gegenstand der abhängigen Ansprüche.

Die Erfindung beruht auf dem allgemeinen Gedanken, einen Kapseläquatorring, welcher nach dem Entfernen einer natürlichen Linse in den eröffneten Kapselsack eines Auges implantierbar ist und im implantierten Zustand mit seinem Außenumfang an der Innenseite des Kapselsacks im wesentlichen an dessen Äquator anliegt und diesen radial stabilisiert, geschlossen und mit mehreren falt- und/oder knickbaren Segmenten sowie steifen Segmenten zu versehen. Dabei sind die falt- und/oder knickbaren Segmente sowie die steifen Segmente in Umfangsrichtung jeweils abwechselnd angeordnet.

Dadurch kann im Vergleich zu einem herkömmlichen, beispielsweise C-förmigen Kapselspannring, bei welchem es durch eine führende Öse zu einer starken Belastung und Dehnung der Zonulafasern im Kapselsackäquator kommt, eine verminderte und intraoperativ individuell ausrichtbare Kapselsackbelastung erreicht werden. Auch das Implantieren des erfindungsgemäßen Kapseläquatorrings selbst ist im Vergleich zum Implantieren eines herkömmlichen, rigiden und offenen C-förmigen Kapselspannringes deutlich gewebeschonender und damit verträglicher. Bei herkömmlichen Kapselspannringen kann es in seltenen Fällen beim Implantieren zu einer Abscherung der Zonulafasern im Implantationsbereich bzw. zum Zug in einem gegenüberliegenden Zonulabereich kommen. Dabei werden die Zonulafasern bei der Implantation des Kapselspannringes sowohl tangential als auch im gegenüberliegenden Bereich stark belastet, was im ungünstigsten Fall zu einer Dislokation des gespannten Spannrings in den Glaskörperraum führt. Eine Explantation ist dadurch erheblich erschwert. Diese Nachteile sind beim erfindungsgemäßen Kapseläquatorring nicht zu befürchten, da dieser sich während des Implantieren langsam und schonend an den Kapselsackäquator anlegt und nach dem Entfernen des Inzisionsinjektors vollständig und selbständig an dem Kapselsackäquator anlegt.

Zum Implantieren des Kapseläquatorrings kann dieser beispielsweise einfach oder doppelt gefaltet sein und sogar problemlos über einen Mikroinzisionsinjektor mit nur 1,4 mm Innenlumendurchmesser implantiert werden. Durch die Doppelfaltung kann eine noch bessere Anpassung der Entfaltung des Kapseläquatorrings von innen nach außen und damit ein noch schonenderes Implantieren erreicht werden. Von besonderem Vorteil ist, dass die Verwendung eines Viskoelastikums zum Implantieren des erfindungsgemäßen Kapseläquatorrings nicht unbedingt erforderlich ist, zur Vermeidung von Luftbläschen aber eingesetzt werden kann.

Darüber hinaus bietet die erfindungsgemäße Lösung den großen Vorteil, dass eine gleichmäßige, da 360° abdeckende, Kapselsackausspannung erreicht werden kann, wobei der Durchmesser des falt- und/oder knickbaren Kapselsackäquatorrings optimal an einen Innendurchmesser des eröffneten Kapselsacks angepasst ist. Dies ist insbesondere im Hinblick auf rotationsstabilisatorische oder individualisierte Intraokularlinsen interessant. Darüber hinaus ist eine Kapselsackschrumpfung im Vergleich zu herkömmlichen, offenen und rigiden Kapselspannringen deutlich reduziert.

Zweckmäßig weist der Kapseläquatorring mehrere Umfangssegmente auf. Als besonders vorteilhaft hat sich hierbei eine Anordnung von insgesamt 16 Umfangssegmenten erwiesen, welche abwechselnd als steife PMMA-Segmente (Polymethylmethacrylat) und flexible, hydrophile HEMA/MMA Copolymer-Segmente (Hydroxyethylmethacrylat-co-Methylmethacrylat), nachfolgend als Copolymer-Segmente bezeichnet, ausgebildet sind. Die segmentartige Ausbildung des Kapseläquatorrings gewährleistet eine gute Falt- bzw. Knickbarkeit und dadurch ein erleichtertes Implantieren des Kapseläquatorrings in den eröffneten Kapselsack.

PMMA ist ein seit langem in der Katarakt- und insbesondere in der Augenchirurgie verwendeter Werkstoff, der einerseits durchsichtig ist, so dass aus diesem Material beispielsweise auch künstliche Linsen hergestellt werden können und andererseits langjährige Erfahrungen im Hinblick auf die Verträglichkeit dieses Materials vorhanden sind. Darüber hinaus bietet PMMA eine gute Eigensteifigkeit sowie ein gutes Formgedächtnis, was dabei hilft, dass sich der implantiere Kapseläquatorring selbständig und exakt im Kapselsack entfaltet. Durch die gute Eigensteifigkeit kann insbesondere eine bessere Widerstandsfähigkeit gegenüber einem Kapselsackschrumpfungsdruck von außen erreicht werden.

Die Copolymer-Segmente hingegen gewährleisten die gute Knick- bzw. Faltbarkeit des Kapseläquatorrings, wobei diese ein Abknicken um bis zu 180° problemlos verkraften. Auch bei dem Copolymer sowie den das Copolymer bildenden Polymeren bestehen langjährige Erfahrungen hinsichtlich seiner Verträglichkeit, so dass der erfindungsgemäße Kapseläquatorring nur Materialien aufweist, welche klinisch langjährig erprobt und bewährt sind.

Vorzugsweise weist der Kapseläquatorring einen an seine Stirnseiten anschließenden scharfkantigen Außenumfang, insbesondere eine scharfkantige anteriore und posteriore Randgestaltung auf. Diese doppelte scharfkantige Randgestaltung ermöglicht insbesondere eine 360°-Barriere zur Nachstarreduktion bereits in der Kapselsackperipherie. Darüber hinaus bewirkt die scharfe Randgestaltung eine Knickung und damit eine Diskontinuität am Kapselsack, die eine Proliferation von Linsenepithelzellen auf dem vom Kapselsack gebildeten Leit- bzw. Gerüstwerk verhindert. Bei In-vitro-Kulturen von Linsenepithelzellen hat sich dabei gezeigt, dass eine Migration dieser Zellen an einer Gefäßwand an winkelförmigen bzw. scharfkantigen geknickten Überhängen zu einer anschließenden Gefäßwand aufgehalten wird.

Bei einer bevorzugten Ausführungsform der erfindungsgemäßen Lösung liegt eine axiale Breite des Außenumfangs des Kapseläquatorrings bei ca. 0,7 mm, wobei die PMMA-Segmente in der Segmentmitte ca. 0,5 mm und die Copolymer-Segmente ca. 0,7 mm breit sind. Die axiale Breite des Kapseläquatorrings am Äquator des Kapselsacks ist unter anderem deshalb vorteilhaft, weil der Kapseläquatorring wirksam in einer Parallellage zur Äquatorebene gehalten bzw. in diese gedrängt wird und damit zu einer gleichförmigen Aufspannung des Kapselsackes am Äquatorbereich führt, ähnlich wie dieses bei einer vorhandenen natürlichen Linse der Fall ist. Darüber hinaus wird einem Kontakt zwischen Vorderkapselblatt und Hinterkapselblatt entgegengewirkt und so der Kapselsack offen gehalten und zugleich eine innenseitige Berührung des Vorderkapselblatts mit dem Hinterkapselblatt verhindert, wodurch eine Reduktion von Kapselsackfibrosen erreicht werden kann.

Zweckmäßig sind die hydrophilen Copolymer-Segmente mit einem Medikament getränkt. Dies bietet insbesondere den Vorteil, dass beispielsweise ein wasserlösliches Medikament in den Copolymer-Segmenten eingelagert werden kann und nach dem Implantieren des Kapseläquatorrings langsam und gleichmäßig abgegeben werden kann. Darüber hinaus ist eine besonders exakte Dosierung der Abgabemenge bzw. eines Abgabezeitraums einstellbar, welcher im Vergleich zu einer herkömmlichen Medikamentengabe einer deutlich geringeren Schwankungsbreite unterliegt. Gleichzeitig ist ein späterer erneuter Eingriff in das Auge zur Medikamentenzugabe nicht erforderlich.

Des Weiteren beruht die Erfindung auf dem allgemeinen Gedanken, einen Kapseläquatorring, welcher nach dem Entfernen einer natürlichen Linse in den eröffneten Kapselsack eines Auges implantierbar ist, zumindest teilweise aus wasseraufnahmefähigem Material auszubilden und mit einem wässrigen bzw. wasserlöslichen Medikament zu tränken. Hierdurch ist es möglich, ein erforderliches Medikament zusammen mit dem Kapseläquatorring im Auge zu platzieren und dadurch eine besonders gleichmäßige und lokale Verteilung des Medikaments zu erreichen. Da die für den erfindungsgemäßen Kapseläquatorring eingesetzten Materialien bereits seit langem klinisch erprobt sind, bestehen hinsichtlich deren Verträglichkeit langjährige Erfahrungen, so dass eine besonders verträgliche Implantation erzielt werden kann. Dabei ist denkbar, dass das zumindest teilweise wasseraufnahmefähige Material, welches mit dem wässrigen bzw. wasserlöslichen Medikament getränkt ist, beispielsweise an einem Innenumfang oder an einem Außenumfang am Kapseläquatorring angeordnet ist. Denkbar ist auch eine lokale oder gänzliche Beschichtung des Kapseläquatorrings mit dem wasseraufnahmefähigen Material. Die Anordnung des wasseraufnahmefähigen und mit Medikamenten getränkten Materials ist dabei sowohl beim erfindungsgemäßen geschlossenen und falt- bzw. knickbaren Kapseläquatorring mit Segmenten als auch bei herkömmlichen, beispielsweise offenen, rigiden und C-förmigen Kapselspannringen möglich.

Weitere wichtige Merkmale und Vorteile der Erfindung ergeben sich aus den Unteransprüchen, aus den Zeichnungen und aus der zugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein bevorzugtes Ausführungsbeispiel der Erfindung ist in den Zeichnungen dargestellt und wird in der nachfolgenden Beschreibung näher erläutert.

Dabei zeigen, jeweils schematisch,

Fig. 1 einen schematisierten Längsschnitt eines Auges mit im eröffneten Kapselsack implantieren Kapseläquatorring,

Fig. 2 einen erfindungsgemäßen Kapseläquatorring in axialer Ansicht,

Fig. 3 eine Darstellung wie in Fig. 2, jedoch bei einer radialen Ansicht.

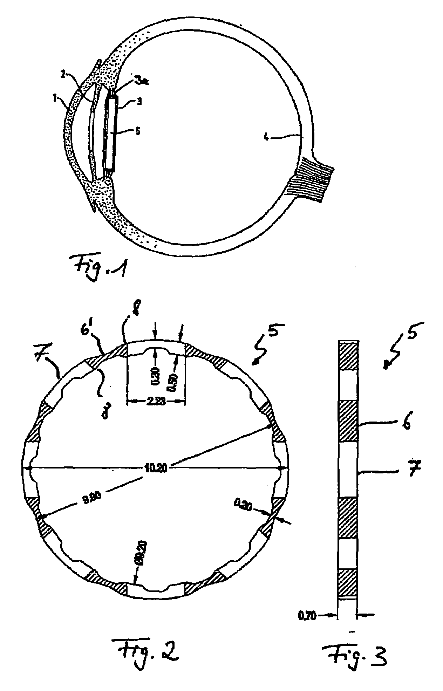

Entsprechend Fig. 1 besitzt das dort dargestellte Auge in bekannter Weise eine Hornhaut 1, eine Iris 2, einen normalerweise die natürliche Linse aufnehmenden Kapselsack 3 sowie eine Retina 4. Im dargestellten Beispiel ist die natürliche Linse entfernt. Dazu ist der Kapselsack 3 auf seiner der Iris 2 zugewandten Seite eröffnet. Bei dieser Operation, beispielsweise einer Kataraktoperation, können Zonulafasern 3a, welche den Kapselsack 3 an seinem Äquator innerhalb des Auges haltern, mehr oder weniger stark beschädigt werden. Um eine damit eingehende Verformungen des Kapselsackes 3 sowie Überlastungen der unbeschädigten Zonulafasern 3a zu vermeiden, kann in den Kapselsack 3 ein erfindungsgemäßer Kapseläquatorring 5 eingesetzt werden. Insbesondere wird ein derartiger Kapseläquatorring 5 intraoperativ bei kompromittierter Zonula 3a zur Reduktion der weiteren Zonulabelastung implantiert.

Gemäß den Fig. 2 und 3 ist der Kapseläquatorring geschlossen ausgebildet und weist mehrere falt- und/oder knickbare Segmente 7 sowie steife Segmente 6 auf, die in Umfangsrichtung des Kapseläquatorrings 5 jeweils abwechselnd angeordnet sind. Vorzugsweise besteht der Kapseläquatorring 5 dabei aus insgesamt 16 Umfangssegmenten 6 und 7, welche abwechselnd als steife PMMA-Segmente 6 (Polymethylmethacrylat) und flexible, hydrophile HEMA/MMA Copolymer-Sergmente 7 (Hydroxyethylmethacrylat-co-Methylmethacrylat) ausgebildet sind. Die steifen PMMA-Segmente 6 sind zur Segmentmitte hin radial zumindest von innen, vorzugsweise aber zusätzlich auch von außen verjüngt und weisen eine hohe Eigensteifigkeit auf, was zu einem verbesserten Formgedächtnis des Kapseläquatorrings 5 führt und darüber hinaus eine erhöhte Widerstandsfähigkeit gegenüber dem Kapselsackschrumpfungsdruck von außen ergibt. Die steifen PMMA-Segmente 6 sind gemäß Fig. 2 und 3 schraffiert dargestellt und zumindest ca. 0,2 mm dick und weisen eine nach innen gerichtete seitliche Übergangszone 8 auf.

Die Copolymer-Segmente 7 verjüngen sich ebenfalls zur Segmentmitte hin radial von innen, wodurch sich bei der axialen Seitenansicht die Form eines Telefonhörers ergibt. Die Copolymer-Segmente 7 weisen beispielsweise einen ca. 28%-igen Wasseranteil auf. Die HEMA-Interponate 7 sind im Vergleich zu den PMMA-Segmenten 6 dicker und in der Mitte etwas verjüngt, um die Faitbarkeit bzw. die Knickbarkeit und die Flexibilität des Ringsystems zu gewährleisten.

Generell weist der Kapseläquatorring 5 einen an seine Stirnseiten anschließenden scharfkantigen Außenumfang, insbesondere eine scharfkantige anteriore und posteriore Randgestaltung auf. Die scharfkantige anteriore und posteriore Randgestaltung sowie die bei ca. 0,7 mm liegende Breite der PMMA-Segmente 6 wirkt einem Kontakt zwischen Vorderkapselblatt und Hinterkapselblatt entgegen und hält dadurch den Kapselsack offen, wodurch eine Reduktion von Kapselsackfibrosen erreicht werden kann. Die doppelte scharfkantige Randgestaltung ermöglicht zudem eine 360°-Barriere zur Nachstarreduktion bereits in der Kapselsackperipherie.

Eine axiale Breite des Außenumfangs des Kapseläquatorrings 5 liegt bei ca. 0,7 mm, wobei die PMMA-Segmente 6 in der Segmentmitte ca. 0,5 mm und die Copolymer-Segmente 7 ca. 0,7 mm breit sind.

Generell können die Kapseläquatorringe 5 mittels Pinzette oder allen verfügbaren Injektorsystemen implantiert werden, so dass kein spezielles Implantationssystem erforderlich ist. Der Kapseläquatorring 5 kann sogar über einen Mikroinzisionsinjektor mit lediglich 1,4 mm Innenlumendurchmesser problemlos implantiert werden, wobei über das gleiche Implantationssystem nachfolgend auch die Intraokularlinse implantiert werden kann.

Der Kapseläquatorring 5 kann einfach oder doppelt gefaltet mittels oben beschriebenem Injektionssystem implantiert werden, wobei eine Doppelfaltung eine bessere Anpassung der späteren Entfaltung des Kapseläquatorringes 5 im Kapselsack 3 von innen nach außen ermöglicht. Für die Implantation des Kapseläquatorrings 5 ist ein Viskoelastikum im Implantationssystem nicht erforderlich, es vermeidet aber das Auftreten von Luftbläschen bei der Implantation. Wird der Kapseläquatorring 5 langsamer implantiert, trifft er bogenförmig geöffnet auf dem gegenüberliegenden Kapselsackäquator, wodurch eine besonders schonende Implantation erreicht werden kann.

Generell kann vorgesehen sein, dass die hydrophilen Copolymer-Segmente 7 mit einem Medikament getränkt sind und dadurch eine Zusatzfunktion aufweisen. Insbesondere kann hierdurch erreicht werden, dass das verabreichte Medikament langsam und in vorab festgelegter Dosierung abgegeben wird, ohne dass eine andere Form der Medikamenteneinnahme erforderlich wäre.

Durch die Implantation des erfindungsgemäßen Kapseläquatorrings 5 kann ein vorbestimmter Kapselsackdurchmesser erreicht werden, was im Hinblick auf rotationsstabilisatorische oder individualisierte Intraokularlinsen interessant erscheint.

Generell sind die Kapseläquatorringe 5 mit unterschiedlichen Durchmessern herstellbar, wobei dieser beispielsweise 10,2 mm betragen kann.

Vorteile des erfindungsgemäßen Kapeläquatorrings 5 sind dabei insbesondere: Eine verminderte individuell ausrichtbare Kapselsackbelastung intraoperativ, eine 360° Nachstarreduktion, ein zirkuläres Offenhalten des Kapselsacks aufgrund der seitlichen Höhe, eine verminderte Vorderkapselfibrose für eine gute Fundusvisualisierung der Netzhautperipherie und eine gleichmäßige Kapselsackausspannung.

Durch die in der Ophtalmologie bereits seit langem eingesetzten Materialien PMMA und HEMA kann eine gute Verträglichkeit derselben gewährleistet werden und dadurch eine besonders schonende und patientenfreundliche Implantation der Kapseläquatorringe 5 erreicht werden.

Unabhängig von der Form bzw. dem Aufbau des Kapseläquatorringes 5 kann dieser zumindest teilweise aus wasseraufnahmefähigem Material bestehen, wobei dieses mit einem wässrigen bzw. wasserlöslichen Medikament getränkt ist. Denkbar sind hierbei insbesondere Beschichtungen von Kapselsackäquatorringen mit einem wasseraufnahmefähigen Material oder die Ausbildung derselben bzw. einzelner Bestandteile davon, wodurch der Kapseläquatorring 5 auch bei anderem Aufbau, beispielsweise als C-förmiger Spannring, als Medikamententräger eingesetzt werden kann.

Da der Kapseläquatorring radial außerhalb des für das Sehen benötigten Linsenbereiches liegt, können eventuelle Verfärbungen des Ringes durch das Medikament ohne weiteres hingenommen werden.

Zusammenfassend lassen sich die wesentlichen Merkmale der erfindungsgemäßen Lösung wie folgt charakterisieren:

Die Erfindung sieht vor, einen Kapseläquatorring 5, welcher nach dem Entfernen einer natürlichen Linse in den eröffneten Kapselsack 3 eines Auges implantierbar ist, geschlossen und mit mehreren falt- und/oder knickbaren Segmenten 7 sowie steifen Segmenten 6 auszubilden, welche in Umfangsrichtung des Kapseläquatorrings 5 jeweils abwechselnd angeordnet sind.

Im Vergleich zu bisherigen offenen und rigiden Spannringen führt die Implantation des erfindungsgemäßen Kapseläquatorrings 5 zu einer verminderten und individuell ausrichtbaren Kapselsackbelastung sowie einer verminderten Vorderkapselfibrose und einer gleichmäßigen Kapselsackausspannung.

## Patentansprüche

1. Kapseläquatorring (5), welcher nach dem Entfernen einer natürlichen Linse in den eröffneten Kapselsack (3) eines Auges implantierbar ist und im implantierten Zustand mit seinem Außenumfang an der Innenseite des Kapselsacks (3) im wesentlichen an dessen Äquator anliegt und den Kapselsack (3) radial stabilisiert,
**dadurch gekennzeichnet,**
**dass** der Kapseläquatorring (5) geschlossen ist und mehrere falt- und/oder knickbare Segmente (7) sowie steife Segmente (6) aufweist, die in Umfangsrichtung jeweils abwechselnd angeordnet sind.

2. Kapseläquatorring nach Anspruch 1,
**dadurch gekennzeichnet,**
**dass** der Kapseläquatorring (5) 16 Umfangssegmente (6, 7) aufweist.

3. Kapseläquatorring nach Anspruch 1 oder 2,
**dadurch gekennzeichnet,**
**dass** die Umfangssegmente (6, 7) abwechselnd als steife PMMA-Segmente (6) (Polymethylmethacrylat) und HEMA/MMA Copolymer-Segmente (7) (Hydroxyethylmethacrylat-co-Methylmethacrylat) ausgebildet sind.

4. Kapseläquatorring nach Anspruch 3,
**dadurch gekennzeichnet,**
**dass** sich die PMMA-Segmente (6) zur Segmentmitte hin radial zumindest von innen verjüngen.

5. Kapseläquatorring nach Anspruch 3 oder 4,
**dadurch gekennzeichnet,**
**dass** sich die PMMA-Segmente (6) zur Segmentmitte hin in Axialrichtung des Ringes (5) verjüngen.

6. Kapseläquatorring nach einem der Ansprüche 3 bis 5,
**dadurch gekennzeichnet,**
**dass** sich die HEMA/MMA Copolymer-Segmente (7) zur Segmentmitte hin radial von innen verjüngen.

7. Kapseläquatorring nach einem der Ansprüche 3 bis 6,
**dadurch gekennzeichnet,**
**dass** die HEMA/MMA Copolymer-Segmente (7) einen ca. 28%-igen Wasseranteil aufweisen.

8. Kapseläquatorring nach einem der Ansprüche 3 bis 7,
**dadurch gekennzeichnet,**
**dass** eine radiale Dicke der PMMA-Segmente (6) in der Segmentmitte ca. 0,2 mm beträgt.

9. Kapseläquatorring nach einem der Ansprüche 3 bis 8,
**dadurch gekennzeichnet,**
**dass** eine axiale Breite des Außenumfangs des Kapseläquatorrings (5) bei ca. 0,7 mm liegt, wobei die PMMA-Segmente (6) in der Segmentmitte ca. 0,5 mm und die HEMA/MMA Copolymer-Segmente (7) ca. 0,7 mm breit sind.

10. Kapseläquatorring nach einem der Ansprüche 1 bis 9,
**dadurch gekennzeichnet,**
**dass** der Kapseläquatorring (5) einen an seine Stirnseiten anschließenden scharfkantigen Außenumfang, insbesondere eine scharfkantige anteriore und posteriore Randgestaltung, aufweist.

11. Kapseläquatorring nach einem der Ansprüche 3 bis 10,
**dadurch gekennzeichnet,**
**dass** die HEMA/MMA Copolymer-Segmente (7) mit einem Medikament getränkt sind.

## Claims

1. A capsular equatorial ring (5) which, after the removal of a natural lens, can be implanted in the opened capsular bag (3) of an eye and, when implanted, rests with its outer periphery against the inside of the capsular bag (3), essentially on the equator thereof, and radially stabilizes the capsular bag (3), **characterized in that** the capsular equatorial ring (5) is closed and has a number of foldable and/or creasable segments (7) and stiff segments (6) that are arranged alternately in the peripheral direction.

2. The capsular equatorial ring as claimed in claim 1, **characterized in that** the capsular equatorial ring (5) has 16 peripheral segments (6, 7).

3. The capsular equatorial ring as claimed in claim 1 or 2, **characterized in that** the peripheral segments (6, 7) are designed alternately as stiff PMMA segments (6) (polymethyl methacrylate) and HEMA/MMA copolymer segments (7) (hydroxyethyl methacrylate-co-methyl methacrylate).

4. The capsular equatorial ring as claimed in claim 3, **characterized in that** the PMMA segments (6) taper radially toward the segment center at least from the inside.

5. The capsular equatorial ring as claimed in claim 3 or 4, **characterized in that** the PMMA segments (6) taper in the axial direction of the ring (5) toward the segment center.

6. The capsular equatorial ring as claimed in one of claims 3 through 5, **characterized in that** the HEMA/MMA copolymer segments (7) taper radially toward the segment center from the inside.

7. The capsular equatorial ring as claimed in one of claims 3 through 6, **characterized in that** the HEMA/MMA copolymer segments (7) have an approximately 28% water content.

8. The capsular equatorial ring as claimed in one of claims 3 through 7, **characterized in that** a radial thickness of the PMMA segments (6) in the segment center is approximately 0.2 mm.

9. The capsular equatorial ring as claimed in one of claims 3 through 8, **characterized in that** an axial width of the outer periphery of the capsular equatorial ring (5) is approximately 0.7 mm, the PMMA segments (6) being approximately 0.5 mm wide in the segment center, and the HEMA/MMA copolymer segments (7) being approximately 0.7 mm wide in the segment center.

10. The capsular equatorial ring as claimed in one of claims 1 through 9, **characterized in that** the capsular equatorial ring (5) has a sharp-edged outer periphery adjoining its end faces, in particular a sharp-edged anterior and posterior configuration.

11. The capsular equatorial ring as claimed in one of claims 3 through 10, **characterized in that** the HEMA/MMA copolymer segments (7) are impregnated with a medicament.

## Revendications

1. Anneau équatorial capsulaire (5), qui peut être implanté après le retrait d'un cristallin naturel dans le sac capsulaire ouvert (3) d'un oeil et s'applique à l'état d'implantation par son pourtour extérieur sur le côté intérieur du sac capsulaire (3), essentiellement sur l'équateur de ce dernier, et stabilisent radialement le sac capsulaire (3), **caractérisé en ce que** l'anneau équatorial capsulaire (5) est fermé et présente plusieurs segments pliables et/ou flexibles (7) ainsi que des segments rigides (6), qui sont disposés en alternance respective dans la direction périphérique.

2. Anneau équatorial capsulaire selon la revendication 1, **caractérisé en ce que** l'anneau équatorial capsulaire (5) comporte 16 segments périphériques (6, 7).

3. Anneau équatorial capsulaire selon l'une des revendications 1 ou 2, **caractérisé en ce que** les segments périphériques (6, 7) sont réalisées en alternance sous forme de segments de PMMA rigides (6) (polyméthyl-méthacrylate) et de segments de copolymère HEMA/MMA (7) (hydroxyéthylméthacrylate-co-méthylméthacrylate).

4. Anneau équatorial capsulaire selon la revendication 3, **caractérisé en ce que** les segments de PMMA (6) se rétrécissent radialement au moins de l'intérieur en direction du centre du segment.

5. Anneau équatorial capsulaire selon l'une des revendications 3 ou 4, **caractérisé en ce que** les segments de PMMA (6) se rétrécissent en direction du centre du segment dans la direction axiale de l'anneau (5).

6. Anneau équatorial capsulaire selon l'une des revendications 3 à 5, **caractérisé en ce que** les segments de copolymère HEMA/MMA (7) se rétrécissent radialement de l'intérieur en direction du centre du segment.

7. Anneau équatorial capsulaire selon l'une des revendications 3 à 6, **caractérisé en ce que** les segments de copolymère HEMA/MMA (7) présentent un pourcentage d'eau d'environ 28%.

8. Anneau équatorial capsulaire selon l'une des revendications 3 à 7, **caractérisé en ce qu'**une épaisseur radiale des segments de PMMA (6) au centre du segment est d'environ 0,2 mm.

9. Anneau équatorial capsulaire selon l'une des revendications 3 à 8, **caractérisé en ce qu'**une largeur axiale du pourtour périphérique de l'anneau équatorial capsulaire (5) est d'environ 0,7 mm, les segments de PMMA (6) ayant au centre du segment une largeur d'environ 0,5 mm et les segments de copolymère HEMA/MMA (7) ayant une largeur de 0,7 mm.

10. Anneau équatorial capsulaire selon l'une des revendications 1 à 9, **caractérisé en ce que** l'anneau équatorial capsulaire (5) présente un pourtour extérieur à bords vifs se raccordant à ses côtés frontaux, en particulier une configuration de bordure antérieure et postérieure à bords vifs.

11. Anneau équatorial capsulaire selon l'une des revendications 3 à 10, **caractérisé en ce que** les segments de copolymère HEMA/MMA (7) sont imprégnés d'un médicament.
